# EUROPEAN PATENT SPECIFICATION

(11) **EP 3 377 278 B1**
(45) Date of publication and mention of the grant of the patent: **06.04.2022**
(21) Application number: 16705646.4
(22) Date of filing: 03.02.2016
(51) Int. Cl.: B25J 9/16, B25J 9/00, A61F 2/70, A61H 3/00, A61B 5/11, A61F 5/01, A61F 2/60

(54) **FALL MITIGATION AND RECOVERY METHODS FOR A LEGGED MOBILITY EXOSKELETON DEVICE**
FALLABFEDERUNGS- UND RÜCKHOLVERFAHREN FÜR EINE EXOSKELETTVORRICHTUNG MIT BEINMOBILITÄT
PROCÉDÉS D'ATTÉNUATION DE CHUTE ET DE RÉCUPÉRATION POUR UN DISPOSITIF D'EXOSQUELETTE DE MOBILITÉ SUR JAMBES

(30) Priority: 16.11.2015 US 201562255549 P
(43) Date of publication of application: 26.09.2018
(73) Proprietor: Parker-Hannifin Corporation, Cleveland, OH 44124 (US)
(72) Inventor: DALLEY, Skyler Ashton, Shaker Heights, Ohio 44120 (US); FARRIS, Ryan, Hartville, Ohio 44632 (US); ETHERIDGE, Steven Jefferson-Shawn, Tallmadge, Ohio 44278 (US)
(74) Representative: Davies, Gregory Mark
(86) International application number: PCT/US2016/016319
(87) International publication number: WO 2017/087016

(56) References cited:
- EP-A1- 1 103 450
- EP-A2- 2 151 374
- WO-A1-2012/094486
- WO-A2-2010/025419
- WO-A2-2011/096965
- US-A1- 2006 011 391
- US-A1- 2010 094 188
- US-A1- 2011 082 566
- US-A1- 2015 190 248

## Description

### Field of Invention

The present invention relates generally to fall mitigation safety methods, including detection, behavior, and recovery, for a legged mobility device or "exoskeleton" device.

### Background of the Invention

There are currently on the order of several hundred thousand spinal cord injured (SCI) individuals in the United States, with roughly 12,000 new injuries sustained each year at an average age of injury of 40.2 years. Of these, approximately 44% (approximately 5300 cases per year) result in paraplegia. One of the most significant impairments resulting from paraplegia is the loss of mobility, particularly given the relatively young age at which such injuries occur. Surveys of users with paraplegia indicate that mobility concerns are among the most prevalent, and that chief among mobility desires is the ability to walk and stand. In addition to impaired mobility, the inability to stand and walk entails severe physiological effects, including muscular atrophy, loss of bone mineral content, frequent skin breakdown problems, increased incidence of urinary tract infection, muscle spasticity, impaired lymphatic and vascular circulation, impaired digestive operation, and reduced respiratory and cardiovascular capacities.

In an effort to restore some degree of legged mobility to individuals with paraplegia, several lower limb orthoses have been developed. The simplest form of such devices is passive orthotics with long-leg braces that incorporate a pair of ankle-foot orthoses (AFOs) to provide support at the ankles, which are coupled with leg braces that lock the knee joints in full extension. The hips are typically stabilized by the tension in the ligaments and musculature on the anterior aspect of the pelvis. Since almost all energy for movement is provided by the upper body, these passive orthoses require considerable upper body strength and a high level of physical exertion, and provide very slow walking speeds.

The hip guidance orthosis (HGO), which is a variation on long-leg braces, incorporates hip joints that rigidly resist hip adduction and abduction, and rigid shoe plates that provide increased center of gravity elevation at toe-off, thus enabling a greater degree of forward progression per stride. Another variation on the long-leg orthosis, the reciprocating gait orthosis (RGO), incorporates a kinematic constraint that links hip flexion of one leg with hip extension of the other, typically by means of a push-pull cable assembly. As with other passive orthoses, the user leans forward against a stability aid (e.g., bracing crutches or a walker) while un-weighting the swing leg and utilizing gravity to provide hip extension of the stance leg. Since motion of the hip joints is reciprocally coupled through the reciprocating mechanism, the gravity-induced hip extension also provides contralateral hip flexion (of the swing leg), such that the stride length of gait is increased. One variation on the RGO incorporates a hydraulic-circuit-based variable coupling between the left and right hip joints. Experiments with this variation indicate improved hip kinematics with the modulated hydraulic coupling.

To decrease the high level of exertion associated with passive orthoses, the use of powered orthoses has been under development, which incorporate actuators and drive motors associated with a power supply to assist with locomotion. These powered orthoses have been shown to increase gait speed and decrease compensatory motions, relative to walking without powered assistance.

One issue with both passive or powered orthoses is that injuries can occur in the event of falling. Depending upon the direction or nature of a fall, the locked or released state of portions of the orthoses can be determinative of the nature or extent of injury. The use of powered orthoses presents an opportunity for electronic control of the orthoses. There now exists, however, no methods that can adequately detect the precise nature of a fall in progress, and adjust the orthoses by locking and/or releasing different portions of the orthoses as may be warranted by a given stage of a fall in progress to mitigate potential injuries from falling.

Examples of powered orthoses are known. WO/2010/044087, US 2010/0094188, and US 8,096,965 disclose a powered exoskeleton bracing system/exoskeleton bracing system. These prior art devices, however, have been insufficient for full protection and control in the event of falling. The conventional methods associated with these devices in particular do not generate alerts in response to falling or changing stance, although these conditions may be indicated. Instead, the safety features generate alerts that tend to be in response to a defective nature or state of the exoskeleton device or its components. Alerts, for example, may be provided as to such conditions as sensor fault(s), detection of "high" temperature(s), detection of battery charger (High Severity Alerts accompanied by Solid Red LEDs), detection of "medium" temperature(s), detection of "critical" battery levels (Medium Severity Alerts accompanied by Flashing Red LEDs), detection of low temperature(s), and detection of "low" battery levels (Low Severity Alerts accompanied by Flashing Yellow LEDs). Detection of High Severity Alerts in particular may result in a control response whereby actuation of the exoskeleton device is halted. Halting actuation, however, can be undesirable in the context of falling depending upon the characteristics of the fall (e.g., the direction, type, or extent of the fall).

There have been attempts to provide at least generalized detection and alerts in connection with falling. For example, US 8,348,875 discloses a method of controlling an exoskeleton bracing system to walk forward comprising operating an alerting device to generate an alert in response to a sensed condition, wherein the sensed condition comprises falling. US 8,905,955 B2 discloses a method of controlling an exoskeleton bracing system comprising halting actuation of the motorized joints when a signal that is received from a tilt sensor indicates falling. These methods are described entirely within the context of standing and sitting transitions. More generally, conventional detection and control methods have proven to be insufficient for full mitigation in response to a sensed fall. Different directions, types, extents, and related fall characteristics are not ascertained with precision by conventional methods, and the conventional methods thus do not provide control of the exoskeleton device for fall mitigation to reduce potential for injury as may occur for any given specific fall, nor enhance recovery from a fall.

WO2012/094486 discloses a stumble detection system for use with a powered artificial leg for identifying whether a stumble event has occurred. The stumble detection system includes an acceleration sensor for providing acceleration data indicative of the magnitude of acceleration of a person's foot, and a detector that determines whether a stumble event has occurred responsive to the acceleration data and provides an output signal.

WO2011/096965 discloses systems and methods for controlling a lower limb device having at least one powered joint. The method includes detecting a stumble event based on one or more sensor signals associated with an overall motion lower limb device, classifying the stumble event based on sensor signals following the sensor signals associated with the stumble event, and selecting a stumble recovery strategy for the lower limb device based on the classification of the stumble event.

US2006/011391 discloses a supplementary support structure which supports the body of a robot.

EP2151374 discloses a motion controlling apparatus and a motion controlling method for a legged mobile robot.

EP1103450 discloses legged mobile robots and methods of controlling operation of the same.

WO2012/094486 discloses a method of controlling a prosthetic device of a user for fall mitigation.

US2010/094188 discloses a method of controlling an exoskeleton device in accordance with the pre-amble of claims 1 and 11.

### Summary of the Invention

The present invention is defined by the subject-matter of the appended claims.

### Brief Description of the Drawings

Fig. 1 is a drawing depicting an exemplary exoskeleton device as being worn by a user.
Fig. 2 is a drawing depicting a perspective view of an exemplary exoskeleton device in a standing position.
Fig. 3 is a drawing depicting a perspective view of the exemplary exoskeleton device in a seated position.
Fig. 4 is a drawing depicting a front view of the exemplary exoskeleton device in a standing position.
Fig. 5 is a drawing depicting a side view of the exemplary exoskeleton device in a standing position.
Fig. 6 is a drawing depicting a back view of the exemplary exoskeleton device in a standing position.
Fig. 7 is a drawing depicting a perspective view of an exemplary thigh assembly having two exemplary actuator cassettes installed therein.
Fig. 8 is a drawing depicting a front exploded view of the exemplary thigh assembly having two exemplary actuator cassettes installed therein.
Fig. 9 is a drawing depicting a perspective exploded view of the exemplary thigh assembly having two exemplary actuator cassettes installed therein.
Fig. 10 is a drawing depicting a top view of an exemplary actuator cassette.
Fig. 11 is a drawing depicting a bottom view of an exemplary actuator cassette.
Fig. 12 is a drawing depicting a perspective view of an exemplary actuator cassette.
Fig. 13 is a drawing depicting a cross-sectional view of an exemplary actuator cassette taken along the longitudinal direction.
Fig. 14 is a drawing depicting a conceptual illustration of fall classification based on direction and extent.
Fig. 15 is a drawing depicting a conceptual illustration of forward fall progression in accordance with embodiments of the present invention.
Fig. 16 is a drawing depicting a conceptual illustration of backward fall progression in accordance with embodiments of the present invention.
Fig. 17 is a drawing depicting a conceptual illustration of prone and kneeling recovery in accordance with embodiments of the present invention.
Fig. 18 is a drawing depicting a conceptual illustration of prone recovery in accordance with embodiments of the present invention.

### Detailed Description

Embodiments of the present invention will now be described with reference to the drawings, wherein like reference numerals are used to refer to like elements throughout. It will be understood that the figures are not necessarily to scale.

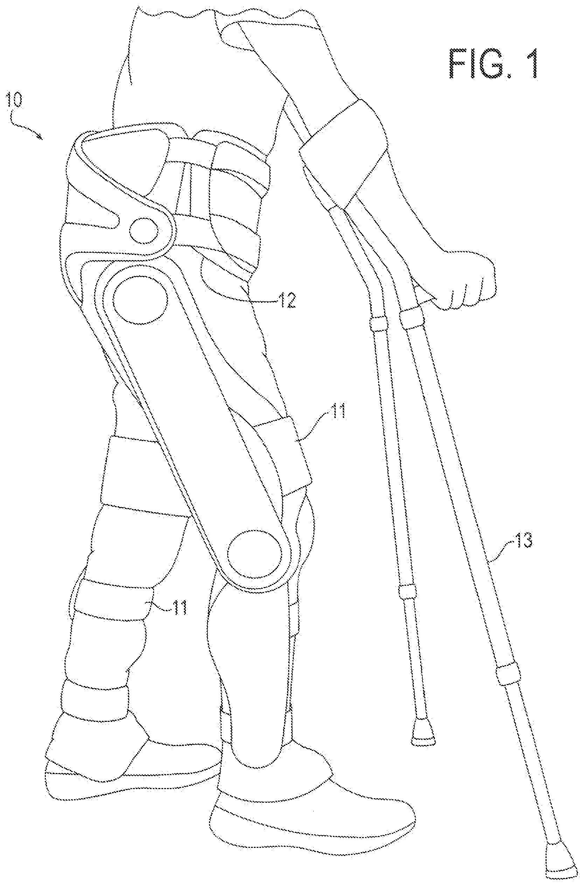

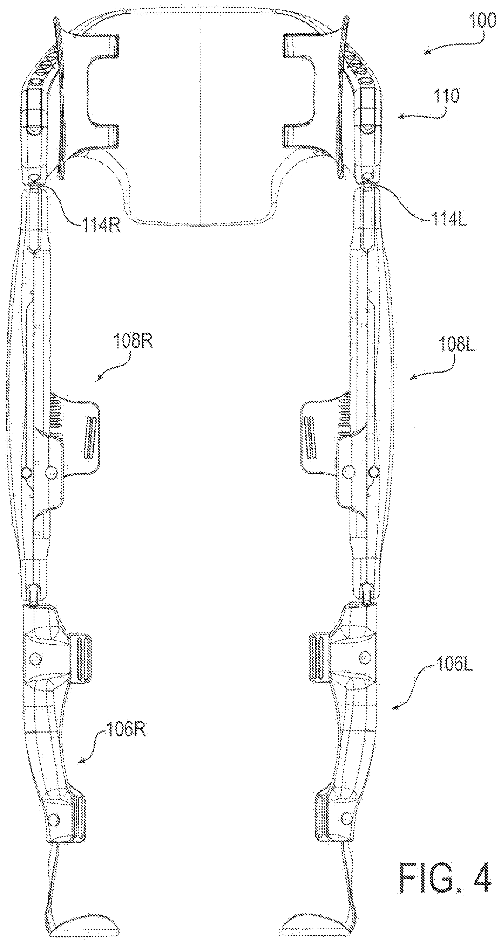

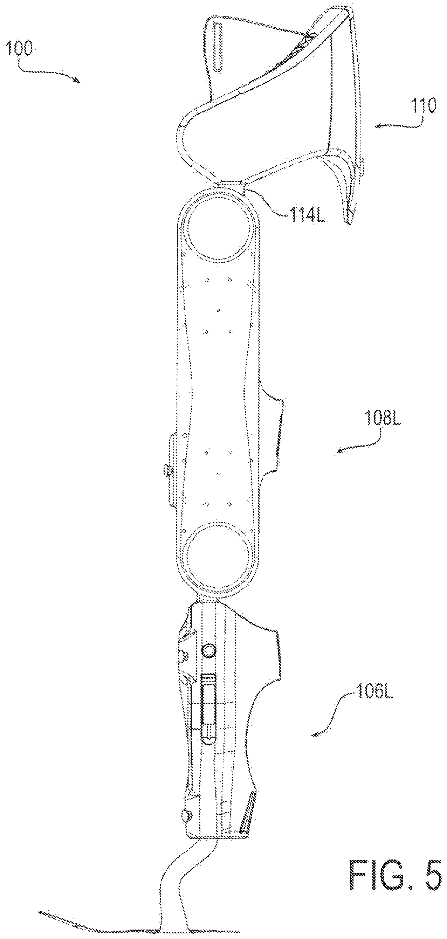

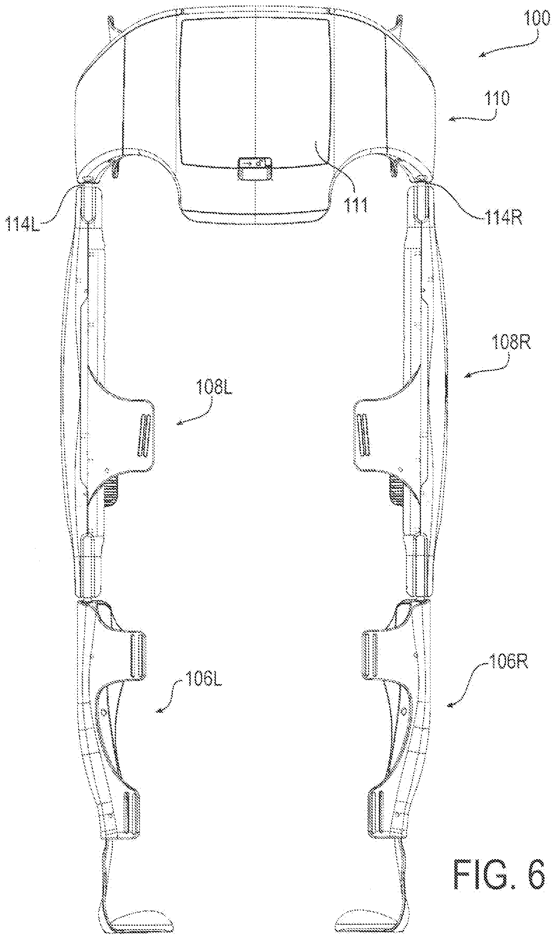

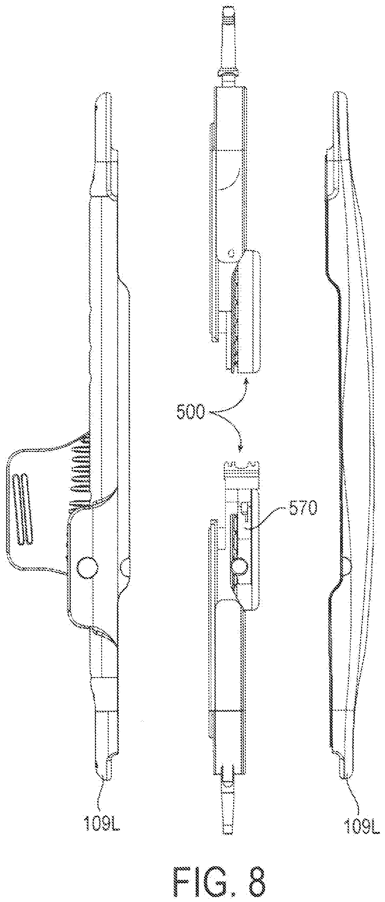

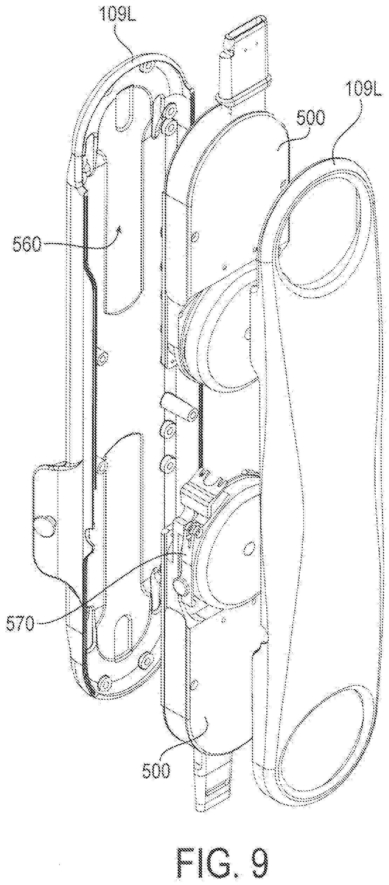

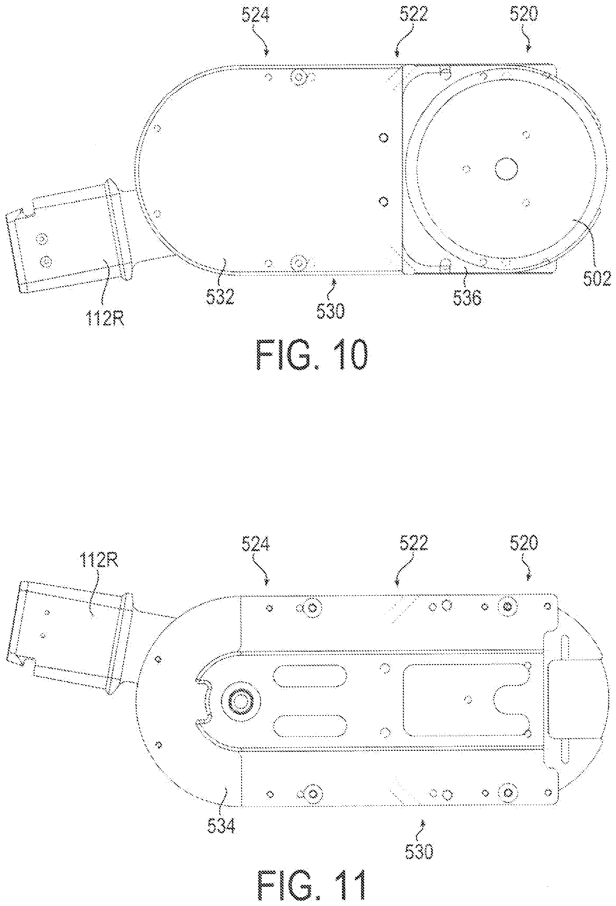

For context, Figs. 1-13 depict various views of an exemplary exoskeleton device that may be used in connection with the fall mitigation and recovery control methods of the present invention. A somewhat generalized description of such exoskeleton device is provided here for illustration purposes. A more detailed description of such device may be found in Applicant's International Patent Appl. No. PCT/US2015/023624 filed on March 3, 2015 and published as WO 2015/153633. It will be appreciated, however, that the described exoskeleton device presents an example usage, and that the fall mitigation and recovery control methods of the present invention are not limited to any particular configuration of an exoskeleton device. Variations may be made to the exoskeleton device, while the features of the present invention remain applicable. In addition, the principles of this invention may be applied generally to orthotic devices, which aid in mobility for persons without use or limited use of a certain body portion. The principles of this invention also may be applied generally to prosthetic devices, which essentially provide an electromechanical replacement of a body part that is not present, such as may be used by an amputee or a person congenitally missing a body portion.

As show in Fig. 1, an exoskeleton device 10, which also may be referred to in the art as a "wearable robotic device", can be worn by a user. To attach the device to the user, the device 10 can include attachment devices 11 for attachment of the device to the user via belts, loops, straps, or the like. Furthermore, for comfort of the user, the device 10 can include padding 12 disposed along any surface likely to come into contact with the user. The device 10 can be used with a stability aid 13, such as crutches, a walker, or the like.

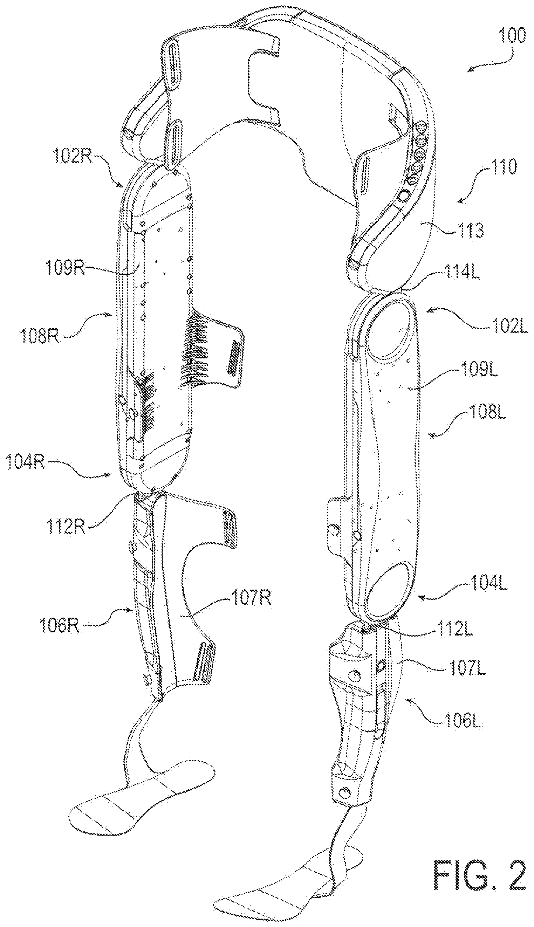

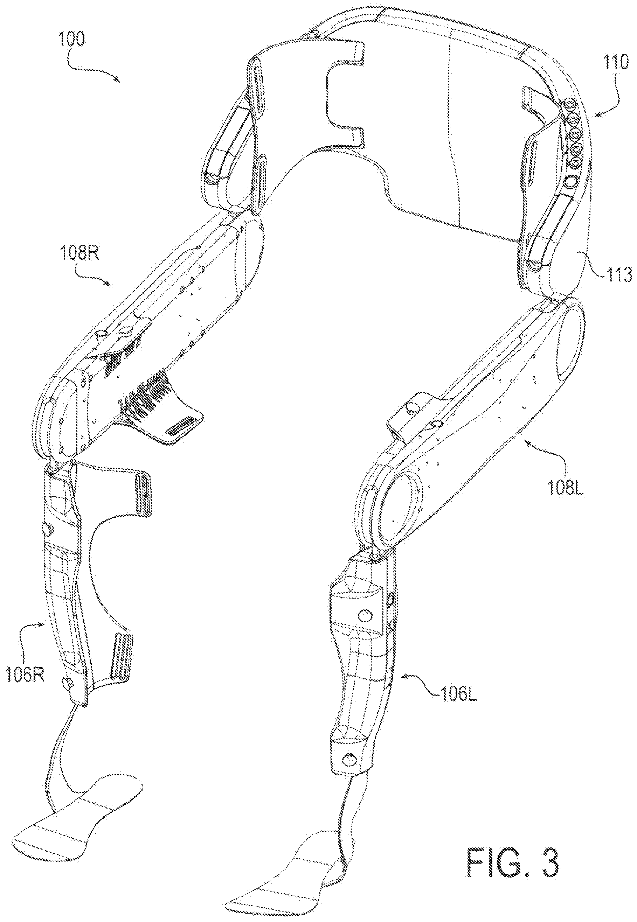

An exemplary legged mobbilty exoskeleton device is illustrated as a powered lower limb orthosis 100 in Figs. 2-6. Specifically, the orthosis 100 shown in Figs. 2-6 may incorporate four drive components configured as electro-motive devices (for example, electric motors), which impose sagittal plane torques at each knee and hip joint components including (right and left) hip joint components 102R, 102L and knee joint components 104R, 104L. Fig. 2 shows the orthosis 100 in a standing position while Fig. 3 shows the orthosis 100 in a seated position.

As seen in the figures, the orthosis contains five assemblies or modules, although one or more of these modules may be omitted and further modules may be added (for example, arm modules), which are: two lower (right and left) leg assemblies (modules) 106R and 106L, two (left and right) thigh assemblies 108R and 108L, and one hip assembly 110. Each thigh assembly 108R and 108L includes a respective thigh assembly housing 109R and 109L, and link, connector, or coupler 112R and 112L extending from each of the knee joints 104R and 104L and configured for moving in accordance with the operation of the knee joints 104R and 104L to provide sagittal plane torque at the knee joints 104R and 104L.

The connectors 112R and 112L further may be configured for releasably mechanically coupling each of thigh assembly 108R and 108L to respective ones of the lower leg assemblies 106R and 106L. Furthermore, each thigh assembly 108R and 108L also includes a link, connector, or coupler 114R and 114L, respectively, extending from each of the hip joint components 102R and 102L and moving in accordance with the operation of the hip joint components 102R and 102L to provide sagittal plane torque at the knee joint components 104R and 104L. The connectors 114R and 114L further may be configured for releasably mechanically coupling each of thigh assemblies 108R and 108L to the hip assembly 110.

In some embodiments, the various components of device 100 can be dimensioned for the user. However, in other embodiments the components can be configured to accommodate a variety of users. For example, in some embodiments one or more extension elements can be disposed between the lower leg assemblies 106R and 106L and the thigh assemblies 108R and 108L to accommodate users with longer limbs. In other configurations, the lengths of the two lower leg assemblies 106R and 106L, two thigh assemblies 108R and 108L, and one hip assembly 110 can be adjustable. That is, thigh assembly housings 109R, 109L, the lower leg assembly housings 107R and 107L for the lower leg assemblies 106R, 106L, respectively, and the hip assembly housing 113 for the hip assembly 110 can be configured to allow the user or medical professional to adjust the length of these components in the field. For example, these components can consist of slidable or movable sections that can be held in one or more positions using screws, clips, or any other types of fasteners. In view of the foregoing, the two lower leg assemblies 106R and 106L, two thigh assemblies 108R and 108L, and one hip assembly 110 can form a modular system allowing for one or more of the components of the orthosis 100 to be selectively replaced and for allowing an orthosis to be created for a user without requiring customized components. Such modularity can also greatly facilitate the procedure for donning and doffing the device.

In orthosis 100, each thigh assembly housing 109R, 109L may include substantially all the drive components for operating and driving corresponding ones of the knee joint components 104R, 104L and the hip joint components 102R, 102L. In particular, each of thigh assembly housings 109R, 109L may include drive components configured as two motive devices (e.g., electric motors) which are used to drive the hip and knee joint component articulations. However, the various embodiments are not limited in this regard, and some drive components can be located in the hip assembly 110 and/or the lower leg assemblies 106R, 106L.

A battery 111 for providing power to the orthosis can be located within hip assembly housing 113 and connectors 114R and 114L can also provide means for connecting the battery 111 to any drive components within either of thigh assemblies 108R and 108L. For example, the connectors 114R and 114L can include wires, contacts, or any other types of electrical elements for electrically connecting battery 111 to electrically powered components in thigh assemblies 108R and 108L. In the various embodiments, the placement of battery 111 is not limited to being within hip assembly housing 113. Rather, the battery can be one or more batteries located within any of the assemblies of orthosis 100.

The referenced drive components may incorporate suitable sensors and related electronic controller or control devices for use in embodiments of the present invention. Embodiments of the present invention involve detecting a direction and an extent of falls through, for example, the use of accelerometers, gyroscopes, inertial measurement, and other sensors to detect and observe the upper leg orientation or angle and angular velocity, and to classify the fall according to direction and extent of the fall. The electronic control device may then selectively control the drive components to modulate the joint components, and particularly the knee and hip joint components, to apply torque, implement locked or released states, or otherwise effect positioning or movement of the joint components for fall mitigation so as to reduce potential for injury as a result of a fall. The electronic control device further may exercise control of the drive components to modulate the joint components for fall recovery to return the user to a desirable position, and particularly standing, following a fall.

To implement the features of the present invention, the electronic control device may include one or processor devices that are configured to execute program code stored on a non-transitory computer readable medium embodying the control methods associated with the present invention. It will be apparent to a person having ordinary skill in the art of computer programming of electronic devices how to program the electronic control device to operate and carry out logical functions associated with present invention. Accordingly, details as to specific programming code have been left out for the sake of brevity. Also, controller functionality could be carried out via dedicated hardware, firmware, software, or any combinations thereof, without departing from the scope of the invention. As will be understood by one of ordinary skill in the art, therefore, the electronic control device may have various implementations. For example, the electronic control device may be configured as any suitable processor device, such as a programmable circuit, integrated circuit, memory and I/O circuits, an application specific integrated circuit, microcontroller, complex programmable logic device, other programmable circuits, or the like. The electronic control device may also include a non-transitory computer readable medium, such as random access memory (RAM), a read-only memory (ROM), an erasable programmable read-only memory (EPROM or Flash memory), or any other suitable medium. Instructions for performing the methods described below may be stored in the non-transitory computer readable medium and executed by the processor device.

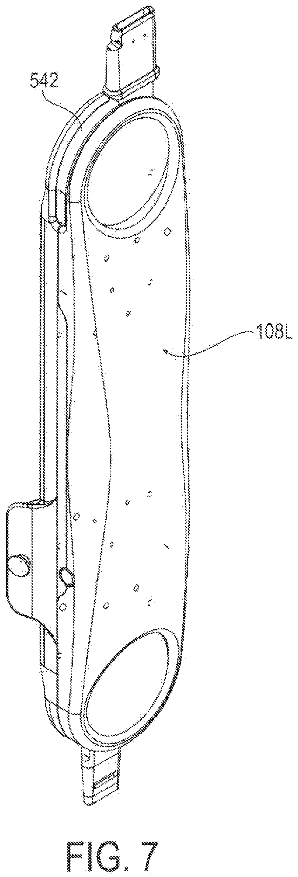

In the various embodiments, to maintain a low weight for orthosis and a reduced profile for the various components, the drive components may include a substantially planar drive system that is used to drive the hip and knee articulations of the joint components. For example, each motor can respectively drive an associated joint component through a speed-reduction transmission using an arrangement of sprocket gears and chains substantially parallel to the plane of sagittal motion. Referring to Figs. 7-13, consolidating the moveable parts into self-contained units, referred to herein as "cassettes," allow for ease of maintenance and replacement because cassettes are swappable, making them easier to service or requiring less of a variety in spare components. As used herein, "self-contained" means that the cassette includes everything necessary to operate in a fully functional manner if supplied with power. Thus, for example, if power is supplied to electrical contacts of the cassette, the cassette would actuate.

In the illustrated embodiment of the drive components, the motor is integrated onto a common baseplate along with sprockets that control the motion of a joint link. Bearings and chains, with and/or without tensioners provide smooth and efficient transfer of motion from the motor to the joint angle. Integrating the motor into the cassette allows for a thinner overall package configuration and provides consistent alignment among parts. Moreover, integrating the motor also creates a larger surface area to transfer and emit heat generated by the motor. In the instance of a mobility assistance device, these cassettes may pertain to a specific joint or set of joints on the device. Each may have a unique actuation unit or share an actuation unit. They may include actuators, with or without a power source, and/or a method of transmitting movement. The illustrated embodiment includes a brushless DC motor with chains and sprockets to create and transmit motion, although other embodiments may utilize electric motors, linear actuators, piezoelectric actuators, belts, ball screws, harmonic drive, gear drive (bevel or planetary), or any combination thereof. The cassettes may also house the electronic control device, and further may contain the referenced sensor elements such as the accelerometers, gyroscopes, inertial measurement, and other sensors to detect and observe the upper leg orientation or angle and angular velocity. The self-contained cassette units can be preassembled to aid in manufacturing the broader device. This allows for quick servicing of the device since individual cassettes can be swapped out and serviced.

Therefore, a removable, self-contained, ovular actuator cassette 500 may be receivable in a receptacle of a wearable robotic device. The cassette 500 may include a first circular portion 520 housing a motive device (e.g., an electric motor) 502. A second circular portion 522 may be longitudinally offset and longitudinally overlapping the first circular portion and may house a first portion of a drivetrain 514, 516 operatively coupled to and driven by the motive device 502. A third circular portion 524 may be longitudinally offset from the first and second circular portions and longitudinally overlapping the second circular portion and may house a second portion of the drivetrain 504. These three overlapping circular portions make an ovular shape, which may include the referenced sensors and electronic control devices. Therefore, an ovular housing 530 may support the motive device 502 and drivetrain 502, 514, 516. Long sides of the ovular housing are straight and parallel with each other and tangentially terminate as curved end surfaces of the ovular housing.

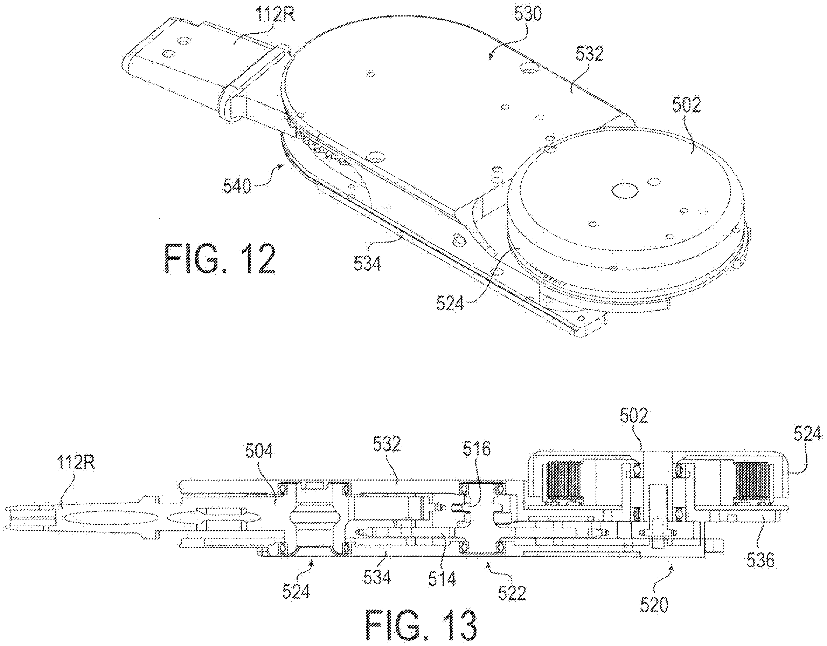

Referring to Figs. 7-13, with Fig. 13 of the right side being representative, the powered joints may be implemented by disposing a joint sprocket gear 504 at one end of thigh assembly housing 109R parallel to the sagittal plane and configuring the joint sprocket gear 504 to rotate parallel to the sagittal plane. To provide the sagittal plane torque for knee joint component 102R, the connector 112R can extend from the joint sprocket gear 504 and be mechanically connected, so that rotation of the joint sprocket gear 504 results in application of torque to the lower leg assembly 106. A slot or receiving element can be provided for the connector 112R to link the thigh assembly 108R and lower leg assembly 106R. The receiving element and the connector 112R can be configured such that the connector can removably connect the thigh assembly 108R and lower leg assembly 106R. In the various embodiments, clips, screws, or any other types of fastener arrangements can be used to provide a permanent or a removable connection. In some embodiments, quick connect or "snap-in" devices can be provided for providing the connection. That is, these quick connect devices allow connections to be made without the need of tools. These types of quick connect devices can not only be used for mechanically coupling, but for electrical coupling with the sensors and control electronics. In some embodiments, a single quick connect device can be used to provide both electrical and mechanical coupling. However, the various embodiments are not limited in this regard and separate quick connect devices can be provided for the electrical and mechanical coupling. It is worth noting that with quick disconnect devices at each joint, the orthosis can be easily separated into three or five modular components - right thigh, left thigh, right lower leg, left lower leg, and hip assemblies - for ease of donning and doffing and also for increased portability.

The knee joint component 104R may be actuated via operation of a motor 502, as discussed above. The motor 502 can be an electric motor that drives the knee joint 104R (i.e., joint sprocket gear 504) using a two-stage chain drive transmission. For example, as shown in Fig. 13, a first stage can include the motor 502 driving, either directly or via a first chain, a first drive sprocket gear 514. The first drive sprocket gear 514 is mechanically coupled to a second drive sprocket gear 516 so that they rotate together about the same axis based on the power applied by motor 502 to first drive sprocket gear 514. The second drive sprocket gear 516 can be arranged so that it is disposed in the same plane as the joint gear 504. Thus, a second chain can then be used to drive joint sprocket gear 504 using the second drive sprocket gear 516 and actuate the knee joint 104R. The gear ratios for the various components described above can be selected based on a needed amount of torque for a joint, power constraints, and space constraints.

Each stage of the chain drive transmission can include tensioners, which can remove slack from a chain and mitigate shock loading. Such tensioners can be adjustable or spring loaded. In addition, a brake 570 can be provided for motor 502. For example, a solenoid brake may be provided which engages a brake pad against the rotor 524 of the motor 502 in one state, and disengages the brake pad in another state. However, the various embodiments are not limited to this particular brake arrangement and any other methods for providing a brake for motor 502 can be used without limitation.

The configuration illustrated in Fig. 13 has been discussed above with respect to an arrangement of sprocket gears and chains. However, the various embodiments are not limited in this regard. That is, any other arrangement of gears, with or without chains, and providing a reduced profile can be used. Furthermore, the various embodiments disclosed herein are not limited to an arrangement of gears and/or chains. For example, in some configurations, a belt and pulley arrangement could be used in place of the chain and sprocket arrangement. Furthermore, a friction drive arrangement can also be used. Also, any combination of the arrangements discussed above can be used as well. Additionally, different joints can employ different arrangements.

In the various embodiments of the drive components, a motor for each of the hip and knee joint components 102R, 102L, 104R, 104L can be configured to provide a baseline amount of continuous torque and a higher amount of torque for shorter periods of time. For example, in one configuration, at least 10 Nm of continuous torque and at least 25 Nm of torque for shorter (i.e., 2-sec) durations are provided. In another example, up to 12 Nm of continuous torque and 40 Nm of torque for shorter (i.e., 2-sec) durations. As a safety measure, both knee joints 104R and 104L can include normally locked brakes, as discussed above, in order to preclude knee buckling in the event of a power failure.

The described exoskeleton device can be controlled in a manner that provides (1) fall mitigation by staged fall progression, and (2) fall recovery. In embodiments of the present invention, detected falls are classified by direction and extent. While conventional configurations have suggested fall detection through observation of force or tilt sensors, classification of the fall according to direction and/or extent has not been employed in connection with fall detection and recovery. Control methods are described for controlling joints and various components of an exoskeleton device based on fall detection and classification for a fall mitigation operation, and further for controlling components of the exoskeleton device to execute a fall recovery operation. Although the exemplary control methods are described below as a specific order of executing functional logic steps, the order of executing the steps may be changed relative to the order described. Also, two or more steps described in succession may be executed concurrently or with partial concurrence. It is understood that all such variations are within the scope of the present invention.

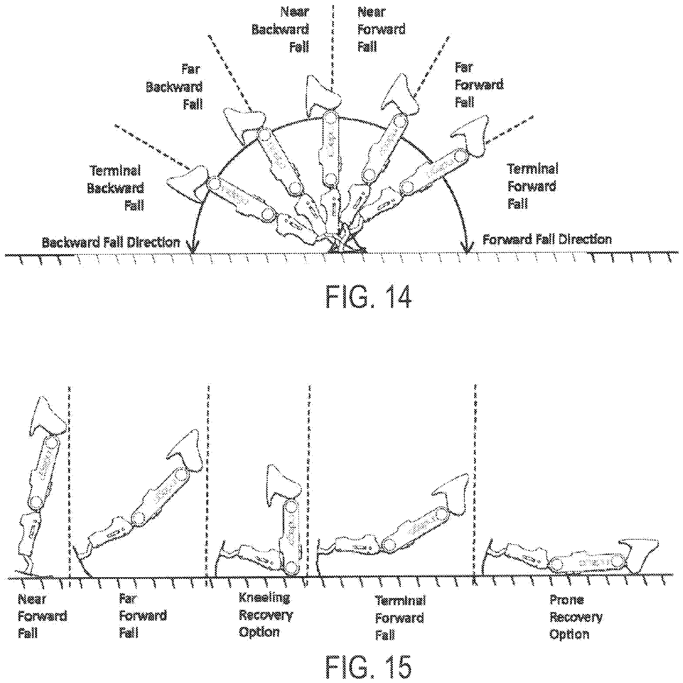

Fig. 14 is a drawing depicting a conceptual illustration of fall classification based on direction and extent. As shown in Fig. 14, falls may be classified according to direction as being either a forward fall or a backward fall. Falls further may be classified according to extent as being near, far, or terminal in accordance with the regions shown in Fig. 14. Sideways falls may be classified similarly based on direction (left or right) and extent (near, far, or terminal). It will be appreciated that the fall classification of Fig. 14 depicts approximate ranges and may be varied depending upon circumstances. In addition, although fall extent in Fig. 14 is broadly classified as near, far, or terminal, intermediate classifications or multiple stages within one or more of the broad categories may be employed in the fall mitigation methods described herein.

An aspect of the present invention includes fall mitigation control methods to generate staged falling control of the joint components, related drive components, and motors of the exoskeleton device, which provide fall mitigation in an attempt to protect the user as the user falls, and/or allow the user to come to an intermediate or terminal position from which the user can recover. In general, the staged falling control methods for fall mitigation may include the steps of: (1) detecting a fall state including a direction and extent of a fall; (2) classifying the fall state based on a direction and an extent of the fall; and (3) controlling the exoskeleton device to selectively modulate exoskeleton components in accordance with the fall classification. The control method may be applied to an exoskeleton device generally including a drive component that drives a joint component, and the control method may include detecting a fall state including a direction and extend of a fall, and controlling the drive component to modulate the joint component to perform a fall mitigation operation. Accordingly, the methods of the present invention may be employed on a relatively simple orthotic device including only one joint component (e.g., a singular knee orthotic), or more complex exoskeleton devices that include a plurality of joint components, such as a legged mobility device having left and right knee and hip joint components such as the exoskeleton device described above. As used throughout, modulating any given joint component or joint components may include applying torque, locking, releasing or otherwise effecting the position or movement of the joint component. In general, the staged falling methods enable the user's torso to remain upright as the user falls. With such control, the user's head is less exposed to the environment because the head will be led to the ground by following either the buttocks or the knees, and thus the head will travel at lower speeds particularly because the upper body is not rotating and other portions of the body absorb initial impacts. This in turn reduces the likelihood and severity of head injury should a fall occur.

The present invention, therefore, provides a method of controlling an orthotic device of a user for fall mitigation. In exemplary embodiments, the orthotic device is an exoskeleton device that is a powered legged mobility device comprising a plurality of drive components that drive joint components including at least knee joint components and hip joint components. The control method may include the steps of: detecting with one or more sensors a fall state including a direction and an extent of a fall; classifying with an electronic control device the fall state based on the direction and the extent of the fall; and controlling the drive components of the exoskeleton device with the electronic control device to selectively modulate the knee and hip joint components in accordance with the fall classification to perform a fall mitigation operation. Additional features of the control methods are described in detail below. Although the exemplary methods may be described below as a specific order of executing functional logic steps, the order of executing the steps may be changed relative to the order described. Also, two or more steps described in succession may be executed concurrently or with partial concurrence. It is understood that all such variations are within the scope of the present invention. The various control methods may be performed by the electronic control device executing program code stored on a non-transitory computer readable medium.

Fig. 15 is a drawing depicting a conceptual illustration of forward fall progression in accordance with embodiments of the present invention. As referenced above, the first and second steps in a staged falling control method may be detecting a fall state and classifying the fall state based on a direction and an extent of the fall. As shown in Fig. 15, in a first example fall mitigation control method, in a typical forward fall progression, a near forward fall would be detected and classified, with the detected direction being forward and the extent determined as being within the near range. Such a classification would be representative of a commonly initiated forward fall. The third general step of a staged falling method may be controlling the exoskeleton device to selectively modulate exoskeleton components in accordance with the fall classification. In exemplary embodiments of the present invention, when the classification step results in a near forward fall classification, the exoskeleton components are controlled by controlling the drive components to (1) apply torque to the knee joint components and the hip joint components such that leg components of the exoskeleton device (and thus the legs of the user) are straightened and brought together, and (2) lock the knee and hip joint components, thereby permitting the user to stand. When the fall stage corresponds to a near fall, the fall angle is minimal, and the system acts in a manner that would permit immediate recovery to standing without continued falling. By controlling the exoskeleton components to straighten and bring the legs together, a user may potentially return to a standing position. Such control operation further ensures that in the event falling continues, the fall should continue in the same forward direction, thereby avoiding falling to the side insofar as a sideways fall becomes substantially less likely if the feet are together as viewed in the sagittal plane.

The staged falling control method steps of detection and classification of falling based on direction and extent are continuously operative. Accordingly, in the event forward falling continues, as shown in Fig. 15 the classification step will next result in a determination of a far forward fall, as the user enters such far range shown in Fig. 15. In exemplary embodiments of the present invention, when the classification step results in a far forward fall classification, the exoskeleton components are controlled by controlling the drive components to release the knee joint components to perform passive or active flexion of the knees. With the release of the knee joint components, the knees are thus permitted to begin to give and buckle. In this manner, the exoskeleton components are controlled to permit passive flexion of the knees, which enables an upright torso. As shown in Fig. 15, under certain circumstances this can permit the user to come to a kneeling position if the user is able to stabilize himself, such as by using a stability aid or the environment. A kneeling position is desirable because, as further detailed below, the user may recover from kneeling to standing. Reaching a kneeling position further is desirable because the potential of injury to the head or torso from further falling from the kneeling position is significantly less than if a user were to fall fully from standing erect, and possibly striking the head or torso on the ground or an object in the environment from the standing position.

Again, the staged falling control method steps of detection and classification of falling based on direction and extent are continuously operative. It may occur that a user is unable to stabilize himself to a kneeling position, and the forward fall continues. Accordingly, in the event forward falling continues, as shown in Fig. 15 the classification step will next result in a determination of a terminal forward fall classification, as the user enters such terminal range shown in Fig 15. In exemplary embodiments of the present invention, when the classification step results in a terminal forward fall, the exoskeleton components are controlled by controlling the drive components to release the various exoskeleton components, including the knee joint components and the hip joint components, thereby permitting the user to fall progressively to a prone position. By releasing the exoskeleton joint components, including the knee joint components and the hip joint components, this allows the user and device to fall more smoothly to the prone position shown in the right-most panel of Fig. 15. When a full fall is imminent at the terminal stage, the user's upper legs are close to horizontal. The exoskeleton components are controlled to enter a default state in which all joints are free to move, so that the exoskeleton device and the user move together to the greatest extent possible as they meet the ground. Given the previous knee flexion permitted during the far forward stage, the user will tend to hit the ground in a progressive manner, with the torso following the knees, and the head following the torso, rather than as a single straight impact with all portions of the body impacting at once. With the progressive impact, the impact force is lessened resulting in a substantially reduced potential for injury.

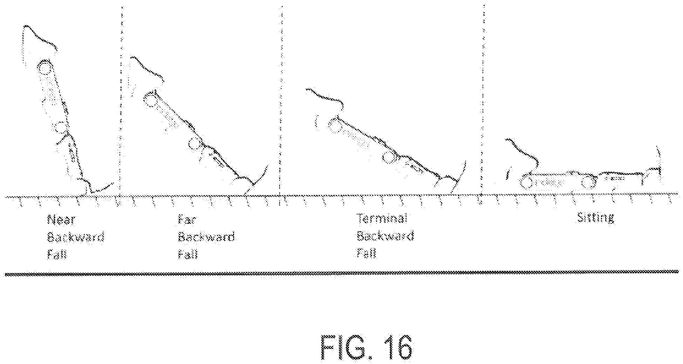

A fall mitigation control method may be performed comparably for a backward fall as performed for a forward fall. Fig. 16 is a drawing depicting a conceptual illustration of backward fall progression in accordance with embodiments of the present invention. As referenced above, the first and second steps in a staged falling control method again may be detecting a fall state and classifying the fall state based on a direction and an extent of the fall. As shown in Fig. 16, in another example control method, in a typical backward fall progression, a near backward fall would be detected and classified, with the detected direction being backward and the extent determined as being within the near range. Such a classification would be representative of a commonly initiated backward fall. The third general step of a staged falling method may be controlling the exoskeleton device to selectively modulate exoskeleton components in accordance with the fall classification. In exemplary embodiments of the present invention, when the classification step results in a near backward fall classification, the exoskeleton components are controlled by controlling the drive components, similarly as in the near forward fall to (1) apply torque to the knee joint components and the hip joint components such that leg components of the exoskeleton device (and thus the legs of the user) are straightened and brought together, and (2) lock the knee and hip joint components, thereby permitting the user to stand. In the backward fall situation as well, when the fall stage corresponds to a near fall, the fall angle is minimal, and the system acts in a manner that would permit immediate recovery to standing without continued falling. Similarly as with the near forward fall, by controlling the exoskeleton components to straighten and bring the legs together during a near backward fall, a user may potentially return to the standing position. Such control operation further ensures that in the event falling continues, the fall should continue in the same backward direction, thereby avoiding falling to the side insofar as a sideways fall becomes substantially less likely if the feet are together as viewed in the sagittal plane. The exoskeleton components further may be controlled to flex the hip joint components during a near backward fall, so that the torso may remain upright in the event the backward fall continues.

Again, the staged falling control method steps of detection and classification of falling based on direction and extent are continuously operative. Accordingly, in the event backward falling continues, as shown in Fig. 16 the classification step will next result in a determination of a far backward fall classification, as the user enters such far range shown in Fig. 16. In exemplary embodiments of the present invention, when the classification step results in a far backward fall classification, the exoskeleton components are controlled to best ensure that the torso remains upright. In particular, the exoskeleton components may be controlled to drive the joint components to perform active flexion of the hip joint components to maintain an upright torso, which ultimately is intended to result in the full fall to a sitting position. concentrating the impact at the buttocks.

Again, the staged falling control method steps of detection and classification of falling based on direction and extent are continuously operative. As the backward falling continues, as shown in Fig. 16 the classification step will next result in a determination of a terminal backward fall classification, as the user enters such terminal range shown in Fig 16. Similarly as with the terminal forward fall, the exoskeleton joints, including the knee joint components and the hip joint components, are released to allow the user and device to fall more smoothly, which in the backward fall situation should result in the user falling to the position shown in the right-most panel of Fig. 16. When a full fall is imminent at the terminal stage, the user's upper legs are close to horizontal. The exoskeleton components are controlled such that the exoskeleton joints enter the referenced default a state in which such joints are free to move, so that the exoskeleton device and the user move together to the greatest extent possible as they meet the ground. With the torso upright from the far fall position, the user will tend to hit the ground largely in a sitting position. Even if the impact were to propel the user backward from sitting, the user again will tend to impact the ground in a progressive manner, with the torso following the buttocks, and the head following the torso, rather than as a single straight impact with all portions of the body impacting at once. In the backward fall also, therefore, with the progressive impact the impact force is lessened resulting in a substantially reduced potential for injury.

The present invention improves over conventional systems, which largely have operated to fully halt actuation in response to detecting a fall. In contrast, in the present invention the exoskeleton components are controlled distinctly at each stage of the fall as detected and classified based on the direction and extent of the fall. In this manner, the exoskeleton motors and related drive components provide active flexion/extension, passive support, or are completely free according to the direction and extent of a fall. The present invention, therefore, provides enhanced opportunity for recovery during or from a fall, and otherwise substantially reduces the potential for injury, as compared to conventional configurations which do not provide for a staged control and fall mitigation based on both direction and extent of a fall.

Enhanced control of recovery after such a staged fall is now described. Should a terminal fall occur, the staged fall control methods described above allow for fall recovery, such that the user may return to a standing position either independently using a stability aid, and/or with the aid of another person. At the outset, recovery control methods are essentially the same in the cases of both a staged forward fall and a staged backward fall, as the different recovery methods would begin from a forward prone position. Accordingly, in the event of a staged backward terminal fall, the first step simply would be for a user to roll over from a sitting or full backward position to a forward prone position. For the initial rollover, the joint components may be driven to release the knee and hip joint components, thereby permitting the user to turn over to the prone position. Once the user has moved to the forward prone position, recovery control follows in a common manner regardless of whether the user initially fell forward or backward.

In exemplary embodiments, the exoskeleton joint components may be controlled to perform a rollover assist operation. For example, the drive components may be controlled to drive the hip and knee joint components to straighten one leg on the side or in the direction of the desired rollover, and optionally further to bend the knee joint component of the other leg. Such leg positioning may provide for an easier rollover by the user.

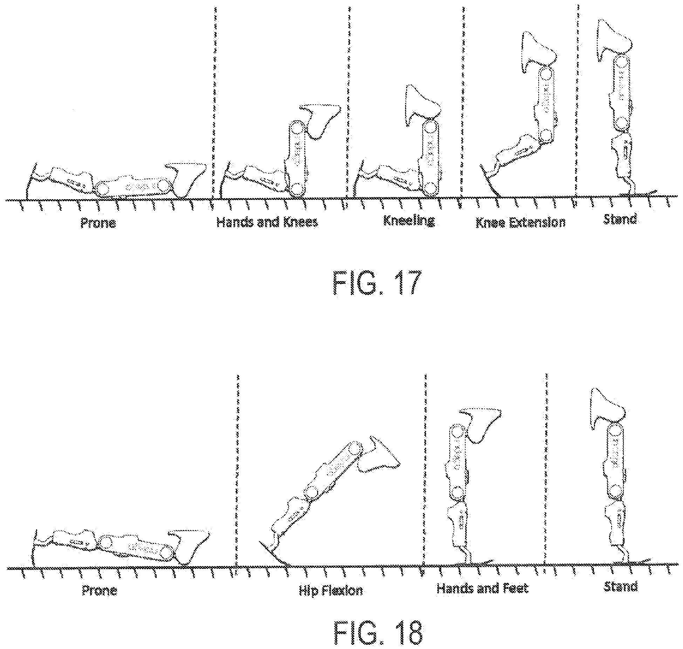

Fig. 17 is a drawing depicting a conceptual illustration of a prone and kneeling recovery control method in accordance with embodiments of the present invention. In exemplary embodiments of the present invention, a recovery control method proceeds as follows. When the user initially is in the forward prone position as referenced above, the exoskeleton components are controlled such that the drive components release the hip and knee joint components (such joints as referenced above may be freed automatically in either a backward or forward terminal fall, or can be freed otherwise while in the forward prone position by putting the device in standby, for instance). With the knee and hip joint components released, the user can then perform a walk-back motion by walking backward with their hands, and in doing so may utilize a stability aid or the environment, to come to their hands and knees. The drive components of the exoskeleton device may or may not be controlled to drive the joint components to assist with this hands walk-back motion. The user then may utilize a stability aid or the environment to straighten the hips and come to a kneeling position. Once the user is kneeling, the exoskeleton components are controlled to provide extensive torque at the knee joints. Such extensive torque tends to straight the leg components of the exoskeleton device, which operates to facilitate standing as the user pushes downward on a stability aid or the environment to stand. As the user stands, the exoskeleton components are controlled to provide support at the hip joint components to keep the torso upright to aid in standing.

As referenced above, there is potential during the staged forward fall for the user coming to the knees during the fall itself, as seen in the third portion of Fig. 15. If the user achieves kneeling during the fall itself, the recovery control method may proceed from the kneeling position without first having to go to the forward prone position. From the kneeling position, as above the exoskeleton components are controlled to provide extensive torque at the knee joints to straighten the leg components to aid in standing, with additional support provided by the hip joint components as the user stands.

In an alternative embodiment of a fall recovery control method, a user may attempt to recover without going to an intermediate kneeling position. Fig. 18 is a drawing depicting a conceptual illustration of prone recovery control method in accordance with embodiments of the present invention. In this alterative fall recovery control method, similarly to the previous method the user begins in the forward prone position with the exoskeleton drive components controlled to release the hip and knee joints (again, such joints may be freed automatically in either a backward or forward terminal fall, or can be freed otherwise while in the forward prone position by putting the device in standby, for instance). After this initial release, the drive components drive the knee joint components to lock in a rigid position. The user then performs a walk-back motion by walking backward with the hands, and in doing so may utilize a stability aid or the environment, to come to the user's hands and feet while the knee joints are controlled to remain rigid as if touching their toes in a conventional exercise manner. The drive components of the exoskeleton device may or may not be controlled further to assist with this hands walk-back motion. As the knees are kept rigid in this embodiment, an intermediate kneeling position is not attained. Following the rigid-knees walk-back, the user utilizes a stability aid or the environment to straighten their hips and stand. Similarly to the previous method, as the user stands, the exoskeleton components are controlled to provide support at the hip joint components to keep the torso upright to aid in standing.

## Claims

1. A method of controlling an exoskeleton device (10) of a user for fall mitigation, the exoskeleton device being a powered legged mobility device comprising a plurality of drive components (502, 514, 516) that drive joint components including at least knee joint (104) components and hip joint (102) components, the control method comprising the steps of:
detecting with one or more sensors a fall state including a direction and an extent of a fall;
classifying with an electronic control device the fall state based on the direction and the extent of the fall; and
controlling the drive components (502, 514, 516) of the exoskeleton device with the electronic control device to selectively modulate the knee and hip joint components in accordance with the fall classification to perform a fall mitigation operation;
**characterized in that** when a fall is detected in a stage that is classified as a near forward or backward fall, the method includes driving the joint components to apply torque to the knee joint (104) components and the hip joint (102) components to straighten and bring the legs of the user are together, to ensure that when the fall continues, the fall continues in a same direction to avoid falling sideways.

2. The method of claim 1, wherein when a fall is detected and classified as a near forward fall, controlling the drive components (502,514, 516) comprises driving the joint components to apply torque to the knee joint (104) components and the hip joint (102) components such that legs of the user are straightened and brought together, and then lock the knee and hip joint components, thereby permitting the user to stand.

3. The method of any of claims 1-2, wherein when a fall is detected and classified as a near backward fall, controlling the drive components (502, 514, 516) comprises driving the joint components to apply torque to the knee joint (104) components and the hip joint (102) components such that legs of the user are straightened and brought together, and then lock the knee and hip joint components, thereby permitting the user to stand.

4. The method of any of claims 1 -3, further comprising the steps of:
when the user is in a kneeling position, controlling the drive components (502, 514, 516) to provide torque at the knee joints (104) for straightening leg components of the exoskeleton device; and
driving the hip joint (102) components to provide support to keep the torso of the user upright, thereby permitting the user to stand.

5. The method of any of claims 1-4, further comprising the steps of:
when the user is in a prone position, controlling the drive components (502, 514, 516) to drive the hip and knee joint (104) components to perform a fall recovery operation from the prone position.

6. The method of claim 5, wherein controlling the drive components (502, 514, 516) to perform the fall recovery operation comprises:
driving the joint components to release the knee and hip joint components, wherein a user performs a walk-back motion including walking backward with the hands and straightening the hips to come to a kneeling position;
providing torque at the knee joints (104) for straightening leg components of the exoskeleton device; and
driving the hip joint (102) components to provide support to keep the torso of the user upright, thereby permitting the user to stand.

7. The method of claim 5, wherein controlling the drive components to perform the fall recovery operation comprises:
driving the joint (104) components to release the knee and hip joint components;
driving the knee joint components to lock the knee joint components, wherein a user performs a walk-back motion including walking backward with the hands with the knees rigid, and
driving the hip joint (102) components to provide support to keep the torso of the user upright, thereby permitting the user to stand.

8. The method of any of claims 6-7, further comprising controlling the drive components (502, 514, 516) to drive the joint components to aid in the walk-back motion.

9. The method of any of claims 4-8, wherein the user initially begins from a backward fall position, the control method comprising driving the joint components to release the knee and hip joint components, thereby permitting the user to turn over to the prone position.

10. A non-transitory computer readable medium storing program code for use in controlling an exoskeleton device of a user for fall mitigation, the exoskeleton device being a powered legged mobility device comprising a plurality of drive components (502, 514, 516) that drive joint components including at least knee joint (104) components and hip joint (102) components, the program code when executed by a computer performing the method of any one of claims 1 to 9.

11. An exoskeleton system comprising:
an exoskeleton device (10) being a powered legged mobility device comprising a plurality of drive components (502, 514, 516) that drive joint components including at least knee joint (104) components and hip joint (102) components;
a plurality of sensors for detecting a fall state including a direction and an extent of a fall; and
an electronic control device configured to classify the fall state based on the direction and the extent of the fall, and control the drive components (502, 514, 516) of the exoskeleton device to selectively modulate the knee and hip joint components in accordance with the fall classification to perform a fall mitigation operation
**characterized in that** when the electronic control device detects a fall in a stage that is classified as a near forward or backward fall, the electronic control device further is configured to drive the joint components to apply torque to the knee joint (104) components and the hip joint (102) components to straighten and bring the legs of the user together, to ensure that when the fall continues, the fall continues in a same direction to avoid falling sideways.

12. The exoskeleton system of claim 11, wherein the electronic control device further is configured to, when the user is in a prone position, control the drive components (502, 514, 516) to drive the hip and knee joint components to perform a fall recovery operation from the prone position.

13. The exoskeleton system of claim 12, wherein the electronic control device is configured to control the drive components (502, 514, 516) to perform the fall recovery operation by:
driving the joint components to release the knee and hip joint components, wherein a user performs a walk-back motion including walking backward with the hands and straightening the hips to come to a kneeling position;
providing torque at the knee joints (104) for straightening leg components of the exoskeleton device; and
driving the hip joint (102) components to provide support to keep the torso of the user upright, thereby permitting the user to stand.

14. The exoskeleton system of claim 12, wherein the electronic control device is configured to control the drive components (502, 514, 516) to perform the fall recovery operation by:
driving the joint components to release the knee and hip joint components; driving the knee joint (104) components to lock the knee joint components, wherein a user performs a walk-back motion including walking backward with the hands with the knees rigid, and
driving the hip joint (102) components to provide support to keep the torso of the user upright, thereby permitting the user to stand.

15. The exoskeleton system of any of claims 13-14, wherein the electronic control device is configured to control the drive components(502, 514, 516) to drive the joint components to aid in the walk-back motion.

## Patentansprüche

1. Verfahren zum Steuern einer Exoskelett-Vorrichtung (10) eines Benutzers zur Sturzabschwächung, wobei die Exoskelett-Vorrichtung eine angetriebene Mobilitätsvorrichtung ist, welche mehrere Antriebskomponenten (502, 514, 516) umfasst, die Gelenkkomponenten antreiben, welche zumindest Kniegelenkkomponenten (104) und Hüftgelenkkomponenten (102) enthalten, wobei das Steuerverfahren die folgenden Schritte umfasst:
Erkennen, mit einem oder mehr Sensoren, eines Sturzzustands, der eine Richtung und ein Ausmaß eines Sturzes beinhaltet;
Klassifizieren, mit einer elektronischen Steuervorrichtung, des Sturzzustands basierend auf der Richtung und dem Ausmaß des Sturzes; und
Steuern der Antriebskomponenten (502, 514, 516) der Exoskelett-Vorrichtung mit der elektronischen Steuervorrichtung zum selektiven Modulieren der Knie- und Hüftgelenkkomponenten gemäß der Sturzklassifizierung zum Ausführen eines Sturzabschwächungsvorgangs;
**dadurch gekennzeichnet, dass**, wenn ein Sturz in einer Phase erkannt wird, die als ein annähernder Vorwärts- oder Rückwärtssturz klassifiziert ist, das Verfahren Antreiben der Gelenkkomponenten zum Ausüben eines Drehmoments auf die Kniegelenkkomponenten (104) und Hüftgelenkkomponenten (102) zum Strecken und Zusammenbringen der Beine des Benutzers beinhaltet, um zu gewährleisten, dass, wenn der Sturz weitergeht, der Sturz in derselben Richtung weitergeht, um Stürzen zur Seite zu vermeiden.

2. Verfahren nach Anspruch 1, wobei, wenn ein Sturz als annähernder Vorwärtssturz erkannt und klassifiziert wird, Steuern der Antriebskomponenten (502, 514, 516) das Antreiben der Gelenkkomponenten zum Ausüben eines Drehmoments auf die Kniegelenkkomponenten (104) und Hüftgelenkkomponenten (102), sodass die Beine des Benutzers gestreckt und zusammengebracht werden, und dann Sperren der Knie- und Hüftgelenkkomponenten umfasst, wodurch es dem Benutzer ermöglicht wird zu stehen.

3. Verfahren nach einem der Ansprüche 1 bis 2, wobei, wenn ein Sturz als annähernder Rückwärtssturz erkannt und klassifiziert wird, Steuern der Antriebskomponenten (502, 514, 516) das Antreiben der Gelenkkomponenten zum Ausüben eines Drehmoments auf die Kniegelenkkomponenten (104) und Hüftgelenkkomponenten (102), sodass die Beine des Benutzers gestreckt und zusammengebracht werden, und dann Sperren der Knie- und Hüftgelenkkomponenten umfasst, wodurch es dem Benutzer ermöglicht wird zu stehen.

4. Verfahren nach einem der Ansprüche 1 bis 3, ferner umfassend die folgenden Schritte:
wenn der Benutzer in einer knienden Position ist, Steuern der Antriebskomponenten (502, 514, 516) zum Vorsehen eines Drehmoments an den Kniegelenken (104) zum Strecken von Beinkomponenten der Exoskelett-Vorrichtung; und
Antreiben der Hüftgelenkkomponenten (102) zum Vorsehen von Unterstützung, um den Rumpf des Benutzers aufrecht zu halten, wodurch es dem Benutzer ermöglicht wird zu stehen.

5. Verfahren nach einem der Ansprüche 1 bis 4, ferner umfassend die folgenden Schritte:
wenn der Benutzer in einer Bauchlage ist, Steuern der Antriebskomponenten (502, 514, 516) zum Antreiben der Hüft- und Kniegelenkkomponenten (104) zum Ausführen eines Sturzrückstellungsvorgangs aus der Bauchlage.

6. Verfahren nach Anspruch 5, wobei das Steuern der Antriebskomponenten (502, 514, 516) zum Ausführen des Sturzrückstellungsvorgangs umfasst:
Antreiben der Gelenkkomponenten zum Freigeben der Knie- und Hüftgelenkkomponenten, wobei ein Benutzer eine Rückwärtslaufbewegung ausführt, die Rückwärtslaufen mit den Händen und Strecken der Hüften beinhaltet, um in eine kniende Position zu gelangen;
Vorsehen eines Drehmoments an den Kniegelenken (104) zum Strecken von Beinkomponenten der Exoskelett-Vorrichtung; und
Antreiben der Hüftgelenkkomponenten (102) zum Vorsehen von Unterstützung, um den Rumpf des Benutzers aufrecht zu halten, wodurch es dem Benutzer ermöglicht wird zu stehen.

7. Verfahren nach Anspruch 5, wobei das Steuern der Antriebskomponenten zum Ausführen des Sturzrückstellungsvorgangs umfasst:
Antreiben der Gelenkkomponenten (104) zum Freigeben der Knie- und Hüftgelenkkomponenten;
Antreiben der Kniegelenkkomponenten zum Sperren der Kniegelenkkomponenten, wobei ein Benutzer eine Rückwärtslaufbewegung ausführt, die Rückwärtslaufen mit den Händen, wobei die Knie starr bleiben, beinhaltet, und
Antreiben der Hüftgelenkkomponenten (102) zum Vorsehen von Unterstützung, um den Rumpf des Benutzers aufrecht zu halten, wodurch es dem Benutzer ermöglicht wird zu stehen.

8. Verfahren nach einem der Ansprüche 6 bis 7, ferner umfassend Steuern der Antriebskomponenten (502, 514, 516) zum Antreiben der Gelenkkomponenten, um bei der Rückwärtslaufbewegung zu unterstützen.

9. Verfahren nach einem der Ansprüche 4 bis 8, wobei der Benutzer anfänglich aus einer Rückwärtssturzposition beginnt, wobei das Steuerverfahren das Antreiben der Gelenkkomponenten zum Freigeben der Knie- und Hüftgelenkkomponenten umfasst, wodurch es dem Benutzer ermöglicht wird, sich in die Bauchlage umzudrehen.

10. Nichtflüchtiges computerlesbares Medium, das Programmcode zum Gebrauch beim Steuern einer Exoskelett-Vorrichtung eines Benutzers zur Sturzabschwächung speichert, wobei die Exoskelett-Vorrichtung eine angetriebene Mobilitätsvorrichtung ist, welche mehrere Antriebskomponenten (502, 514, 516) umfasst, die Gelenkkomponenten antreiben, welche zumindest Kniegelenkkomponenten (104) und Hüftgelenkkomponenten (102) enthalten, wobei der Programmcode, wenn er durch einen Computer ausgeführt wird, das Verfahren nach einem der Ansprüche 1 bis 9 ausführt.

11. Exoskelett-System, umfassend:
eine Exoskelett-Vorrichtung (10), die eine angetriebene Mobilitätsvorrichtung ist, welche mehrere Antriebskomponenten (502, 514, 516) umfasst, die Gelenkkomponenten antreiben, welche zumindest Kniegelenkkomponenten (104) und Hüftgelenkkomponenten (102) enthalten;
mehrere Sensoren zum Erkennen eines Sturzzustands, der eine Richtung und ein Ausmaß eines Sturzes beinhaltet;
eine elektronische Steuervorrichtung, die zum Klassifizieren des Sturzzustands basierend auf der Richtung und dem Ausmaß des Sturzes und zum Steuern der Antriebskomponenten (502, 514, 516) der Exoskelett-Vorrichtung zum selektiven Modulieren der Knie- und Hüftgelenkkomponenten gemäß der Sturzklassifizierung zum Ausführen eines Sturzabschwächungsvorgangs konfiguriert ist;
**dadurch gekennzeichnet, dass**, wenn ein Sturz in einer Phase erkannt wird, die als ein annähernder Vorwärts- oder Rückwärtssturz klassifiziert ist, die elektronische Steuervorrichtung zum Antreiben der Gelenkkomponenten zum Ausüben eines Drehmoments auf die Kniegelenkkomponenten (104) und Hüftgelenkkomponenten (102) zum Strecken und Zusammenbringen der Beine des Benutzers konfiguriert ist, um zu gewährleisten, dass, wenn der Sturz weitergeht, der Sturz in derselben Richtung weitergeht, um Stürzen zur Seite zu vermeiden.

12. Exoskelett-System nach Anspruch 11, wobei die elektronische Steuervorrichtung, wenn der Benutzer in einer Bauchlage ist, ferner zum Steuern der Antriebskomponenten (502, 514, 516) zum Antreiben der Hüft- und Kniegelenkkomponenten zum Ausführen eines Sturzrückstellungsvorgangs aus der Bauchlage konfiguriert ist.

13. Exoskelett-System nach Anspruch 12, wobei die elektronische Steuervorrichtung zum Steuern der Antriebskomponenten (502, 514, 516) zum Ausführen des Sturzrückstellungsvorgangs durch Folgendes konfiguriert ist:
Antreiben der Gelenkkomponenten zum Freigeben der Knie- und Hüftgelenkkomponenten, wobei ein Benutzer eine Rückwärtslaufbewegung ausführt, die Rückwärtslaufen mit den Händen und Strecken der Hüften beinhaltet, um in eine kniende Position zu gelangen;
Vorsehen eines Drehmoments an den Kniegelenken (104) zum Strecken von Beinkomponenten der Exoskelett-Vorrichtung; und
Antreiben der Hüftgelenkkomponenten (102) zum Vorsehen von Unterstützung, um den Rumpf des Benutzers aufrecht zu halten, wodurch es dem Benutzer ermöglicht wird zu stehen.

14. Exoskelett-System nach Anspruch 12, wobei die elektronische Steuervorrichtung zum Steuern der Antriebskomponenten (502, 514, 516) zum Ausführen des Sturzrückstellungsvorgangs durch Folgendes konfiguriert ist:
Antreiben der Gelenkkomponenten zum Freigeben der Knie- und Hüftgelenkkomponenten;
Antreiben der Kniegelenkkomponenten (104) zum Sperren der Kniegelenkkomponenten, wobei ein Benutzer eine Rückwärtslaufbewegung ausführt, die Rückwärtslaufen mit den Händen, wobei die Knie starr bleiben, beinhaltet, und
Antreiben der Hüftgelenkkomponenten (102) zum Vorsehen von Unterstützung, um den Rumpf des Benutzers aufrecht zu halten, wodurch es dem Benutzer ermöglicht wird zu stehen.

15. Exoskelett-System nach einem der Ansprüche 13 bis 14, wobei die elektronische Steuervorrichtung zum Steuern der Antriebskomponenten (502, 514, 516) zum Antreiben der Gelenkkomponenten konfiguriert ist, um bei der Rückwärtslaufbewegung zu unterstützen.

## Revendications

1. Procédé de commande d'un dispositif d'exosquelette (10) d'un utilisateur pour l'atténuation de chute, le dispositif d'exosquelette étant un dispositif de mobilité de jambes motorisés comprenant une pluralité de composants d'entraînement (502, 514, 516) qui entraînent des composants d'articulation comprenant au moins des composants d'articulation du genou (104) et des composants d'articulation de la hanche (102), le procédé de commande comprenant les étapes de :
détection, avec un ou plusieurs capteur(s), d'un état de chute comprenant une direction et une ampleur d'une chute ;
classification, avec un dispositif de commande électronique de l'état de chute sur la base de la direction et de l'ampleur de la chute ; et
commande des composants d'entraînement (502, 514, 516) du dispositif d'exosquelette avec le dispositif de commande électronique pour moduler sélectivement les composants d'articulations du genou et de la hanche conformément à la classification de la chute pour effectuer une opération d'atténuation de la chute ;
**caractérisé en ce que** lorsqu'une chute est détectée dans un stade qui est classée comme une chute proche vers l'avant ou vers l'arrière, le procédé comprend l'entraînement des composants d'articulation pour appliquer un couple aux composants d'articulation du genou (104) et aux composants d'articulation de la hanche (102) pour redresser et rapprocher les jambes de l'utilisateur, pour s'assurer que lorsque la chute continue, la chute continue dans une même direction pour éviter de tomber sur le côté.

2. Procédé selon la revendication 1, dans lequel lorsqu'une chute est détectée et classée comme une chute proche vers l'avant, la commande des composants d'entraînement (502, 514, 516) comprend l'entraînement des composants d'articulation pour appliquer un couple aux composants d'articulation du genou (104) et aux composants d'articulation de la hanche (102) de sorte que les jambes de l'utilisateur soient redressées et rapprochées, et ensuite verrouiller les composants d'articulations du genou et de la hanche, permettant ainsi à l'utilisateur de se tenir debout.

3. Procédé selon l'une des revendications 1 et 2, dans lequel lorsqu'une chute est détectée et classée comme une chute proche vers l'arrière, la commande des composants d'entraînement (502, 514, 516) comprend l'entraînement des composants d'articulation pour appliquer un couple aux composants d'articulation du genou (104) et aux composants d'articulation de la hanche (102) de sorte que les jambes de l'utilisateur soient redressées et rapprochées, et ensuite verrouiller les composants d'articulations du genou et de la hanche, permettant ainsi à l'utilisateur de se tenir debout.

4. Procédé selon l'une des revendications 1 à 3, comprenant en outre les étapes de :
lorsque l'utilisateur est en position agenouillée, commande des composants d'entraînement (502, 514, 516) pour fournir un couple aux articulations du genou (104) pour redresser les composants de jambe du dispositif d'exosquelette ; et
entraînement des composants d'articulation de la hanche (102) pour fournir un support pour maintenir le torse de l'utilisateur droit, permettant ainsi à l'utilisateur de se tenir debout.

5. Procédé selon l'une des revendications 1 à 4, comprenant en outre les étapes de :
lorsque l'utilisateur est en position couchée, commande des composants d'entraînement (502, 514, 516) pour entraîner les composants d'articulations de la hanche et du genou (104) pour effectuer une opération de récupération de chute à partir de la position couchée.

6. Procédé selon la revendication 5, dans lequel la commande des composants d'entraînement (502, 514, 516) pour effectuer l'opération de récupération de chute comprend :
l'entraînement des composants d'articulation pour libérer les composants d'articulations du genou et de la hanche, dans lequel un utilisateur effectue un mouvement de marche arrière comprenant une marche arrière avec les mains et un redressement des hanches pour arriver à une position agenouillée ;
la fourniture d'un couple aux articulations du genou (104) pour redresser les composants de jambe du dispositif d'exosquelette ; et
l'entraînement des composants d'articulation de la hanche (102) pour fournir un support pour maintenir le torse de l'utilisateur droit, permettant ainsi à l'utilisateur de se tenir debout.

7. Procédé selon la revendication 5, dans lequel la commande des composants d'entraînement pour effectuer l'opération de récupération de chute comprend :
l'entraînement des composants d'articulation (104) pour libérer les composants d'articulation du genou et de la hanche ;
l'entraînement des composants d'articulation du genou pour verrouiller les composants d'articulation du genou, dans lequel un utilisateur effectue un mouvement de marche arrière comprenant une marche arrière avec les mains avec les genoux rigides, et
l'entraînement des composants d'articulation de la hanche (102) pour fournir un support pour maintenir le torse de l'utilisateur droit, permettant ainsi à l'utilisateur de se tenir debout.

8. Procédé selon l'une des revendications 6 et 7, comprenant en outre la commande des composants d'entraînement (502, 514, 516) pour entraîner les composants d'articulation afin de faciliter le mouvement de marche arrière.

9. Procédé selon l'une des revendications 4 à 8, dans lequel l'utilisateur commence initialement à partir d'une position de chute vers l'arrière, le procédé de commande comprenant l'entraînement des composants d'articulation pour libérer les composants d'articulations du genou et de la hanche, permettant ainsi à l'utilisateur de retourner à la position couchée.

10. Support non transitoire lisible par ordinateur stockant un code de programme pour une utilisation dans la commande d'un dispositif d'exosquelette d'un utilisateur pour l'atténuation de chute, le dispositif d'exosquelette étant un dispositif de mobilité sur jambes motorisé comprenant une pluralité de composants d'entraînement (502, 514, 516) qui entraînent des composants d'articulation comprenant au moins des composants d'articulation du genou (104) et des composants d'articulation de la hanche (102), le code de programme lorsqu'il est exécuté par un ordinateur effectuant le procédé selon l'une quelconque des revendications 1 à 9.

11. Système d'exosquelette comprenant :
un dispositif d'exosquelette (10) étant un dispositif de mobilité sur jambes motorisé comprenant une pluralité de composants d'entraînement (502, 514, 516) qui entraînent des composants d'articulation comprenant au moins des composants d'articulation du genou (104) et des composants d'articulation de la hanche (102) ;
une pluralité de capteurs pour détecter un état de chute comprenant une direction et une ampleur d'une chute ; et
un dispositif de commande électronique configuré pour classer l'état de chute sur la base de la direction et de l'ampleur de la chute, et commander les composants d'entraînement (502, 514, 516) du dispositif d'exosquelette pour moduler sélectivement les composants d'articulations du genou et de la hanche conformément à la classification de la chute pour effectuer une opération d'atténuation de chute
**caractérisé en ce que** lorsque le dispositif de commande électronique détecte une chute dans un stade qui est classée comme une chute proche vers l'avant ou vers l'arrière, le dispositif de commande électronique est en outre configuré pour entraîner les composants d'articulation pour appliquer un couple aux composants d'articulation du genou (104) et aux composants d'articulation de la hanche (102) pour redresser et rapprocher les jambes de l'utilisateur, pour s'assurer que lorsque la chute continue, la chute continue dans une même direction pour éviter de tomber sur le côté.

12. Système d'exosquelette selon la revendication 11, dans lequel le dispositif de commande électronique est en outre configuré pour, lorsque l'utilisateur est en position couchée, commander les composants d'entraînement (502, 514, 516) pour entraîner les composants d'articulations de la hanche et du genou pour effectuer une opération de récupération de chute à partir de la position couchée.

13. Système d'exosquelette selon la revendication 12, dans lequel le dispositif de commande électronique est configuré pour commander les composants d'entraînement (502, 514, 516) pour effectuer l'opération de récupération de chute par :
entraînement des composants d'articulation pour libérer les composants d'articulations du genou et de la hanche, dans lequel un utilisateur effectue un mouvement de marche arrière comprenant une marche arrière avec les mains et un redressement des hanches pour arriver à une position agenouillée ;
fourniture d'un couple aux articulations du genou (104) pour redresser les composants de jambe du dispositif d'exosquelette ; et
entraînement des composants d'articulation de la hanche (102) pour fournir un support pour maintenir le torse de l'utilisateur droit, permettant ainsi à l'utilisateur de se tenir debout.

14. Système d'exosquelette selon la revendication 12, dans lequel le dispositif de commande électronique est configuré pour commander les composants d'entraînement (502, 514, 516) pour effectuer l'opération de récupération de chute par :
entraînement des composants d'articulation pour libérer les composants d'articulations du genou et de la hanche ;
entraînement des composants d'articulation du genou (104) pour verrouiller les composants d'articulation du genou, dans lequel un utilisateur effectue un mouvement de marche arrière comprenant une marche arrière avec les mains avec les genoux rigides, et
entraînement des composants d'articulation de la hanche (102) pour fournir un support pour maintenir le torse du l'utilisateur droit, permettant ainsi à l'utilisateur de se tenir debout.

15. Système d'exosquelette selon l'une des revendications 13 et 14, dans lequel le dispositif de commande électronique est configuré pour commander les composants d'entraînement (502, 514, 516) pour entraîner les composants d'articulation afin de faciliter le mouvement de marche arrière.
